# EUROPEAN PATENT APPLICATION

(11) **EP 2 829 249 A1**
(43) Date of publication of application: **28.01.2015**
(21) Application number: 13765068.5
(22) Date of filing: 22.03.2013
(51) Int. Cl.: A61B 18/14, A61B 18/12, A61M 25/01

(54) **ABLATION ELECTRODE AND PERFUSION ELECTRODE CATHETER USING SAME**

(30) Priority: 23.03.2012 CN 201210079501
(71) Applicant: Synaptic Medical (Beijing) Co. Ltd., Beijing 100111 (CN)
(72) Inventor: HUA, Xin, Beijing 100111 (CN); HANSEN, Sophia Wang, Beijing 100111 (CN); FENG, Ji, Beijing 100111 (CN); GONG, Jie, Beijing 100111 (CN)
(74) Representative: Schmidt, Steffen J.
(86) International application number: PCT/CN2013/000335
(87) International publication number: WO 2013/139178

(57) **Abstract**

The present invention discloses an ablation electrode used in a catheter. The ablation electrode includes an electrode shell, an optional cavity inside the electrode shell and a temperature sensor. An outflow liquid pathway for the perfusion liquid is arranged on the electrode shell, and an inflow liquid pathway for the perfusion liquid is arranged in the proximal end of the electrode shell. A thermal conduction isolation structure is arranged between the temperature sensor and the liquid pathway. The thickness of the electrode shell where the temperature sensor is located is less than 0.2mm. The present invention also provides a perfusion electrode catheter which includes the ablation electrode. The perfused electrode catheter of the present invention can provide efficient prompts on the rising ablation temperature in the ablation process.

## Description

### Cross Reference to Related Applications

The present application claims the priority of Chinese patent application 201210079501.4 filed on March 23, 2012, the entire contents of which are herein incorporated by reference.

### Field of the Invention

The present invention relates to an ablation electrode and a perfused electrode catheter using the electrode, and more particularly relates to an ablation electrode with a temperature sensor and a perfused electrode catheter using the same.

### Background of the Invention

Electrode catheters have been widely applied to clinical practice for many years. They may be used in hearts to map and stimulate electrical activities and perform ablation therapy on positions occurring abnormal electrical activities.

In clinical use, an electrode catheter enters the body through a main vein or artery, e.g. through a femoral vein, and then is guided into the concerned heart chamber. In some applications, the catheter is expected to have the capability of injecting and/or extracting liquid, and such a function may be completed by a perfused catheter.

In a specific application example, an ablation injury is generated in the heart through catheter intracardiac ablation, so as to cut off an abnormal electrocardio conduction path in the heart. A typical ablation operation process includes that, a catheter provided with an ablation electrode at the distal end thereof is inserted into the heart chamber, a reference electrode is provided and generally attached and fixed to the skin surface of a patient, and a radio frequency (RF) voltage is applied between the ablation electrode and the reference electrode to generate RF electric current flowing through media therebetween, including blood and tissue. The distribution of the electric current depends on the ratio of the contact area of the ablation electrode and the tissue to the contact area of the ablation electrode and the blood. After the heart tissue is sufficiently heated by the heating effect of the RF electric current, tissue cells are destroyed to form an injury in the heart tissue, and the injured tissue does not form electrical conduction. In this process, the heated tissue simultaneously heats the ablation electrode through heat conduction. If the temperature of the electrode is high enough, e.g. higher than 60 °C, a thin film formed by dehydration of blood protein may be formed on the surface of the electrode, and if the temperature is further raised, the dehydrated protein layer is gradually thickened, and thus blood coagulation occurs on the surface of the electrode. Because the electrical conductivity of the dehydrated biomaterial is lower than that of the intracardiac tissue, the impedance for the RF electric current flowing into the tissue is increased, and when the impedance becomes high up to a certain degree, the catheter must be taken out of the body to clean the ablation electrode.

Another method for achieving the aforementioned desired effect is to perfuse the ablation electrode. For example, the ablation electrode is actively cooled by adopting normal saline at room temperature, rather than depending on the relatively passive cooling effect of the blood. Under such a condition, the electrode is effectively cooled, and the surface temperature thereof is no longer the main factor of producing impedance increase and even blood coagulation, so that the intensity of the RF electric current is no longer limited by the surface temperature, the electric current may be increased, and thus a larger injury closer to a sphere shape is formed, generally in a size of 10mm to 12mm.

United States Patents US5, 643,197 and US5, 462,521 disclose a perfused electrode made of a porous material, wherein the electrode has a metallic structure formed by sintering tiny particles, and the liquid cooling of the electrode structure is more effective through multiple interconnected channels. Moreover, the porous structure enables liquid flow to form a liquid film uniformly distributed on the surface of the electrode and becoming a blocking layer between the blood and the surface of the electrode.

Therefore, a novel perfused catheter is needed.

### Summary of the Invention

The present invention provides an ablation electrode for a catheter, including an electrode shell, and an optional cavity and a temperature sensor in the electrode shell; a liquid passage for outflow of perfusion liquid is provided in the electrode shell, and a liquid passage for inflow of the perfusion liquid is provided in the proximal end of the electrode shell; and a heat-conducting insulation structure is provided between the temperature sensor and the liquid passages, and the thickness of the electrode shell where the temperature sensor is located is less than 0.2mm.

The term "optional" used in the context expresses the meaning of "not indispensable" or "not essential". For example, "optional cavity" indicates that there may be or may not be the cavity. This may be selected by those skilled in the art according to conditions.

In an implementation of the present invention, the electrode shell is provided with a through hole, and the temperature sensor is provided in the through hole.

In a specific implementation of the present invention, an insert is provided at the proximal end of the electrode shell, the distal end of the insert extends into the cavity, and the insert includes at least one through hole; the distal end of the through hole in the insert extends into the through hole of the electrode shell, the proximal end of the through hole in the insert is opened at the proximal end of the insert, the distal end of the through hole in the insert is opened on the outer surface of the electrode shell and is flush with the outer surface of the electrode shell, and the temperature sensor is provided at the distal end of the through hole in the insert.

The diameter of the through hole in the electrode shell may be less than 1mm; and preferably, the diameter of the through hole in the electrode shell is less than 0.5mm.

In a specific implementation of the present invention, an insert is provided at the proximal end of the electrode shell, the distal end of the insert extends into the cavity, and the insert includes at least one through hole; the distal end of the through hole in the insert extends to the inner surface of the electrode shell and is flush with the inner surface of the electrode shell, or the distal end of the through hole in the insert partially extends into the electrode shell; the proximal end of the through hole in the insert is opened at the proximal end of the insert, the distal end of the through hole in the insert is opened or has a closed construction, and the temperature sensor is provided at the distal end of the through hole in the insert; and preferably, the thickness of the electrode shell where the temperature sensor is located is less than 0.1 mm.

Preferably, the heat-conducting insulation structure is a nonmetallic heat insulation layer.

The nonmetallic heat insulation layer may be provided on the inner wall and/or outer wall of the through hole in the insert; preferably, the inner wall and/or outer wall of the through hole in the insert is partially or completely provided with the nonmetallic heat insulation layer.

The distance between a temperature sensing portion of the temperature sensor and the top end of the temperature sensor may be less than 0.5mm; preferably, the distance between the temperature sensing portion of the temperature sensor and the top end of the temperature sensor is less than 0.2mm; and more preferably, the distance between the temperature sensing portion of the temperature sensor and the top end of the temperature sensor is less than 0.1 mm.

The nonmetallic heat insulation layer may be made of a high polymer material or ceramic.

The present invention provides a perfused electrode catheter, which is characterized by including:
a catheter main body having a proximal end, a distal end and a central chamber penetrating through the catheter main body;
an ablation portion, comprising a section of elastic tip tube, and having a proximal end, a distal end and at least one chamber penetrating through ablation portion, the proximal end of the ablation portion is fixedly connected with the distal end of the catheter main body;
an ablation electrode, fixedly connected to the distal end of the ablation portion, and including an electrode shell, and an optional cavity and a temperature sensor in the electrode shell, wherein a liquid passage for outflow of perfusion liquid is provided in the electrode shell, and a liquid passage for inflow of the perfusion liquid is provided in the proximal end of the electrode shell; and a heat-conducting insulation structure is provided between the temperature sensor and the liquid passages, and the thickness of the electrode shell where the temperature sensor is located is less than 0.2mm;
a perfusion passage, having a proximal end and a distal end, , the distal end of the perfusion passage extends into the chamber of the ablation portion through the central chamber of the catheter main body and is communicated with the liquid passage of the ablation electrode.

In an implementation of the present invention, the electrode shell is provided with a through hole, and the temperature sensor is provided in the through hole.

In a specific implementation of the present invention, an insert is provided at the proximal end of the electrode shell, the distal end of the insert extends into the cavity, and the insert includes at least one through hole; the distal end of the through hole in the insert extends into the through hole of the electrode shell, the proximal end of the through hole in the insert is opened at the proximal end of the insert, the distal end of the through hole in the insert is opened on the outer surface of the electrode shell and is flush with the outer surface of the electrode shell, and the temperature sensor is provided at the distal end of the through hole in the insert.

The diameter of the through hole in the electrode shell may be less than 1mm; and preferably, the diameter of the through hole in the electrode shell is less than 0.5mm.

In a specific implementation of the present invention, an insert is provided at the proximal end of the electrode shell, the distal end of the insert extends into the cavity, and the insert includes at least one through hole; the distal end of the through hole in the insert extends to the inner surface of the electrode shell and is flush with the inner surface of the electrode shell, or he distal end of the through hole in the insert partially extends into the electrode shell; the proximal end of the through hole in the insert is opened at the proximal end of the insert, the distal end of the through hole in the insert is opened or has a closed construction, and the temperature sensor is provided at the distal end of the through hole in the insert; and preferably, the thickness of the electrode shell where the temperature sensor is located is less than 0.1mm.

A perfusion pipeline is provided in the perfusion passage, and the distal end of the perfusion pipeline may extend to the distal end of the chamber of the ablation portion through a control handle and the central chamber of the catheter main body and is communicated with the liquid passage of the ablation electrode, or extend to the distal end of the central chamber of the catheter main body through the control handle and is communicated with at least one chamber penetrating through the ablation portion. Preferably, the heat-conducting insulation structure is a nonmetallic heat insulation layer.

The nonmetallic heat insulation layer may be provided on the inner wall and/or outer wall of the through hole in the insert; preferably, the inner wall and/or outer wall of the through hole in the insert is partially or completely provided with the nonmetallic heat insulation layer.

The distance between a temperature sensing portion of the temperature sensor and the top end of the temperature sensor may be less than 0.5mm; preferably, the distance between the temperature sensing portion of the temperature sensor and the top end of the temperature sensor is less than 0.2mm; and more preferably, the distance between the temperature sensing portion of the temperature sensor and the top end of the temperature sensor is less than 0.1 mm.

The nonmetallic heat insulation layer may be made of a high polymer material or ceramic.

In a preferred implementation of the present invention, the perfused electrode catheter includes the heat-conducting insulation structure, so that the temperature sensor is separated from the cooling effect of the perfusion liquid, the cooling effect of the perfusion liquid on the part where the temperature sensor is located is weakened, and the heating effect of circumferential heat conduction from the outer wall of the electrode shell on the part where the temperature sensor is located is maintained; and the temperature sensor is provided on the inner surface of the electrode shell or in the through hole of the electrode shell or partially extends into the electrode shell, so that the temperature sensor may detect more temperature rise produced by the heating effect of radio-frequency electric currents.

### Brief Description of the Drawings

Fig. 1 is a structural schematic diagram of a perfused electrode catheter according to the present invention;
Fig. 2 shows a sectional view of a catheter main body 12 according to a preferable implementation of the present invention, illustrating the connection relationship between the catheter main body 12 and an ablation portion 13;
Fig. 3 shows a sectional view along line A-A of Fig. 1;
Fig. 4 shows a sectional view along line B-B of Fig. 3;
Fig. 5 shows a sectional view along line C-C of Fig. 3;
Fig. 6 shows a schematic diagram of a temperature sensor 33 in an ablation electrode 17 according to a preferable implementation of the present invention;
Fig. 7 shows a sectional view of an ablation electrode 17 according to a further preferable implementation of the present invention;
Fig. 8 shows a structural diagram of an ablation electrode 17 according to a further preferable implementation of the present invention;
Fig. 9 shows a sectional view of the ablation electrode 17 shown in Fig. 8;
Fig. 10 shows a sectional view of an ablation electrode 17 according to a further preferable implementation of the present invention;
Fig. 11 shows a sectional view of the ablation electrode 17 shown in Fig. 10;
Fig. 12 shows a sectional view of an ablation electrode 17 according to a further preferable implementation of the present invention;
Fig. 13 shows a sectional view of an ablation electrode 17 according to a further preferable implementation of the present invention; and
Fig. 14 shows a sectional view of an ablation electrode 17 according to a further preferable implementation of the present invention.

### Detailed Description of the Embodiments

The technical solution of the present invention will be further described below in detail with reference to the implementations and in combination with the accompanying drawings, but the present invention is not merely limited to the following implementations.

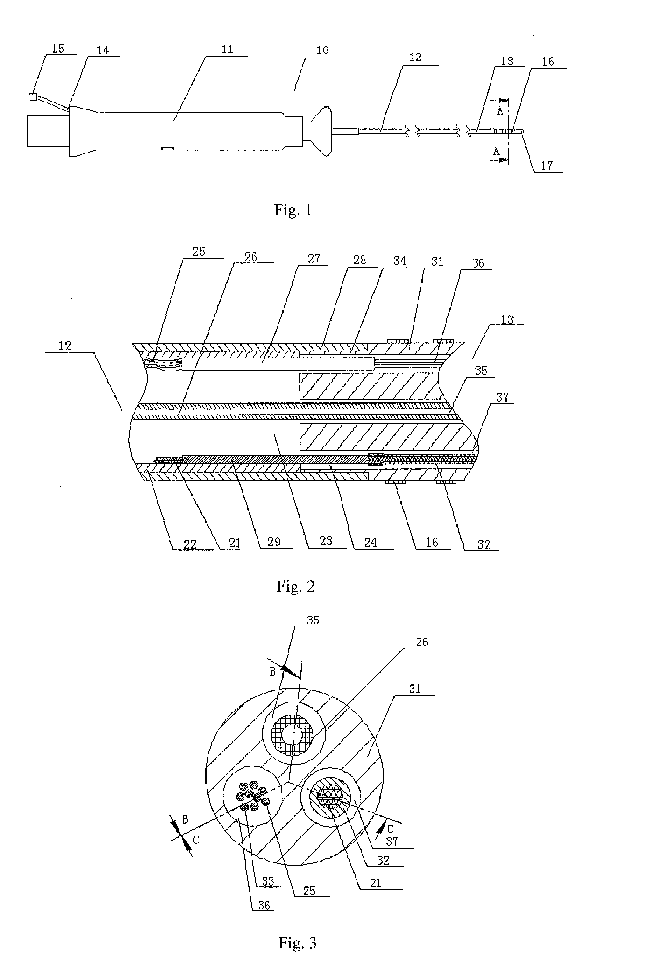

Fig. 1 shows a structural schematic diagram of a perfused electrode catheter 10 with a temperature sensor of the present invention, which includes a catheter main body 12 having a distal end, at which an ablation portion 13 is provided, and a proximal end, at which a control handle 11 is provided.

Fig. 2 shows a sectional view of the catheter main body 12 according to one implementation of the present invention, illustrating the connection relationship between the catheter main body 12 and the ablation portion 13. The catheter main body 12 includes a reinforcement tube 22 and a main body tube 28 sleeved outside the reinforcement tube, wherein the main body tube 28 may be made of a biocompatible high polymer material, e.g. polyether block amide, polyurethane or nylon material, and the wall of the main body tube 28 preferably includes therein at least one metal wire braided layer (not shown in the figure), which may be a stainless steel wire braided layer. There may be one, two or more metal wire braided layers. The reinforcement tube 22 includes a central chamber 23 and may be made of any suitable high polymer material, such as being made by extrusion molding of polyether block amide, polyurethane, polyimide or nylon material. The catheter main body is preferably elongated and bendable, but typically incompressible in the length direction thereof. The central chamber 23 extends in the axial direction of the catheter main body 12; and a conducting wire 25, a traction wire 21 and a perfusion pipeline 26 extend inside the central chamber 23.

The ablation portion 13 includes an elastic tip tube 31, which may be made of a biocompatible material and includes a distal end, a proximal end and at least one chamber, wherein the chamber may be a central or eccentric chamber. In one specific implementation of the present invention, as shown in Fig.2, the elastic tip tube 31 includes a first eccentric chamber 35, a second eccentric chamber 36 and a third eccentric chamber 37. Preferably, the wall of the elastic tip tube 31 includes therein at least one metal wire braided layer (not shown in the figure), which may be a stainless steel wire braided layer. There may be one, two or more metal wire braided layers. Preferably, the proximal end of the elastic tip tube 31 is a thinned end 34 having an outer diameter matched with the inner diameter of the catheter main body 12, as shown in Fig.2. The thinned end 34 is inserted into the catheter main body 12 and may be fixed by bonding, welding or other appropriate means. For example, the thinned end 34 is bonded and fixed to the catheter main body 12 through UV cured adhesive.

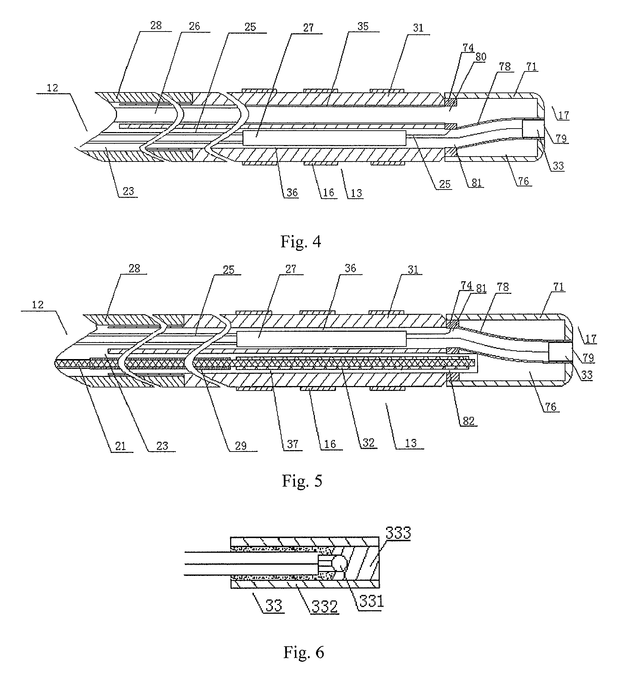

Fig. 3 shows a sectional view along line A-A of Fig. 1, Fig. 4 shows a sectional view along line B-B of Fig. 3, Fig. 5 shows a sectional view along line C-C of Fig. 3, and Fig. 6 shows a schematic diagram of a temperature sensor 33 shown in Fig. 4 and Fig. 5. An ablation electrode 17 is disposed at the distal end of the elastic tip tube 31, and ring electrode(s) 16, the number of which may vary according to actual needs, are provided in the length direction of the elastic tip tube 31. There may be no ring electrode, or there may be one, two, three, four or more ring electrodes. The ablation electrode 17 includes an electrode shell 71 and a cavity 76 therein. The electrode shell 71 is further provided with a through hole 79 having a diameter of less than 1mm. Preferably, the through hole 79 has a diameter of less than 0.5mm. The outer surface of the electrode shell 71 is a part which can be in contact with tissue to be ablated; and the cavity inside the electrode shell 71 is a part which cannot be in contact with the tissue.

In a preferable implementation, as shown in Fig. 4 and Fig. 5, an insert 74 is provided in the cavity 76, wherein the insert 74 is provided at the proximal end of the electrode shell 71, and the distal end of the insert 74 extends into the cavity 76. The insert 74 may be cylindrical, disc-shaped or in other suitable shape and at least includes a through hole 81. The distal end of the through hole 81 extends into the through hole 79 of the electrode shell 71. The extending section of the through hole 81 and the insert 74 may be formed integrally or separately. For example, a hollow tube is inserted into the through hole 81, or the distal end of the through hole 81 is connected with a pipeline or other suitable structure. The proximal end of the through hole 81 is opened at the proximal end of the insert 74, and the distal end of the through hole 81 is opened on the outer surface of the electrode shell 71 and is flush with the outer surface of the electrode shell 71. A temperature sensor 33 is provided at the distal end of the through hole 81 and is fixed by adhesive bonding. The temperature sensor 33 is located at the through hole in the electrode shell, where the thickness of the electrode shell is zero. The through hole 81 may be located close to the axis of the electrode shell 71 or close to the side wall of the electrode shell 71. The insert further includes a through hole 80, and the through hole 80 is a liquid passage for the inflow of perfusion liquid.

As shown in Fig. 6, an outer sleeve 332 is sleeved outside the temperature sensor 33 and the outer sleeve is made of an insulating material or an insulating and thermal insulation material. The distance between a temperature sensing portion 331 of the temperature sensor 33 and the top end of the temperature sensor is less than 0.5mm. Preferably, the distance between the temperature sensing portion of the temperature sensor and the top end of the temperature sensor is less than 0.2mm. More preferably, the distance between the temperature sensing portion of the temperature sensor and the top end of the temperature sensor is less than 0.1mm. Insulating but heat-conducting glue 333 may be provided between the temperature sensing portion 331 and the top end, so as to improve the sensitivity of the temperature sensor and provide enough insulating property at the same time. The top end of the temperature sensor 33 may be flush with the outer surface of the electrode shell or located nearby the outer surface of the electrode shell. For example, a small part of the top end of the temperature sensor 33 protrudes out of the outer surface or retracts inside the outer surface.

The insert 74 further includes a blind hole 82 in which a traction wire 21 is fixed. The through hole 80, the through hole 81 and the blind hole 82 are communicated with the first eccentric chamber 35, the second eccentric chamber 36 and the third eccentric chamber 37 of the elastic tip tube 31 respectively. Preferably, a hollow tube, which may be made of a suitable high polymer material or metallic material, e.g. polyimide or stainless steel, may be welded or bonded on each of the three holes 80, 81 and 82 of the insert 74 respectively.

A heat-conducting insulation structure is provided between the temperature sensor 33 and the liquid passage, and the heat-conducting insulation structure is a nonmetallic heat insulation layer 78, which is disposed on the through hole 81. When the extending section of the through hole 81 and the insert 74 are formed integrally and the insert 74 is made of a metallic material, the nonmetallic heat insulation layer 78 is disposed on the inner wall and/or outer wall of the through hole 81. The inner wall and/or outer wall of the through hole 81 in the context may refer to the inner wall and/or outer wall of a section of the through hole 81 extending in the cavity, or the inner wall and/or outer wall of the entire through hole 81. The nonmetallic heat insulation layer 78 may be a tubular heat insulation layer inserted into the inner wall and/or onto the outer wall of the through hole 81, or may be a nonmetallic heat insulation layer coated on the inner wall and/or outer wall of the through hole 81. When the insert 74 is made of a nonmetallic material, the through hole 81 itself may be used as a nonmetallic heat insulation layer. When the extending section of the through hole 81 and the insert 74 are formed separately and the extending section of the through hole 81 is made of the metallic material, the nonmetallic heat insulation layer 78 is disposed on the inner wall and/or outer wall of the through hole 81. When the extending section of the through hole 81 of the insert is made of a nonmetallic material, the through hole 81 itself may be used as a nonmetallic heat insulation layer. The nonmetallic heat insulation layer 78 may completely or partially cover the inner wall and/or outer wall of the through hole 81. When the nonmetallic heat insulation layer 78 partially covers the inner wall and/or outer wall of the through hole 81, the degree of heat insulation may be adjusted, so as to adjust the temperature detected by the temperature sensor 33. When the material or thickness of the nonmetallic heat insulation layer is changed, the degree of heat insulation may also be adjusted. The nonmetallic heat insulation layer 78 is made of a nonmetallic material with a heat insulation function, which may be a high polymer material or ceramic or other suitable nonmetallic material. The nonmetallic heat insulation layer 78 may be made of a nonmetallic material with high temperature resistance. Because the nonmetallic heat insulation layer 78 is provided on the through hole 81, the temperature sensor is separated from the cooling effect of the perfusion liquid. The cooling effect of the perfusion liquid on the part where the temperature sensor is located is weakened, and the temperature sensor will be able to detect more temperature rise produced by the heating effect of radio-frequency currents.

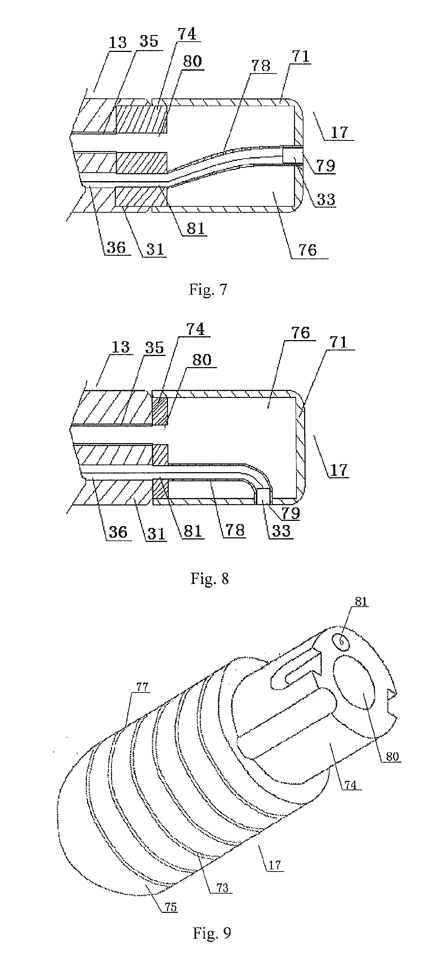

The insert 74 may be completely placed in the cavity of the electrode shell 71, or partially placed in the cavity of the electrode shell 71, as shown in Fig. 7.

The temperature sensor 33 may be a thermocouple, a thermistor or other device. There may be one, two, three or more temperature sensors. As shown in Fig. 4, the distal end of the temperature sensor 33 extends into the second eccentric chamber 36 of the elastic tip tube 31 through the central chamber 23 of the catheter main body 12, then extends into the through hole 81, and is fixed at the distal end of the through hole 81 by glue bonding. The proximal end of the temperature sensor 33 extends into the control handle 11 through the central chamber 23, then extends out of the control handle 11 and is connected with a temperature monitoring device (not shown in the figure).

The electrode shell 71 is provided with a plurality of small holes (not shown in the figure) serving as liquid passages for outflow of the perfumed liquid. The small holes may be formed in any suitable manner. They may be formed in a manner of machining, laser processing or electro-machining and the like, or may also be formed by using a porous material.

A perfusion passage has a proximal end and a distal end, wherein the distal end of the perfusion passage extends into the first eccentric chamber 35 of the elastic tip tube 31 through the control handle 11 and the central chamber 23 of the catheter main body 12 and is communicated with the liquid passage of the ablation electrode 17. The perfusion pipeline 26 is provided in the perfusion passage. The distal end of the perfusion pipeline 26 may extend to the distal end of the first eccentric chamber 35 of the elastic tip tube 31 through the control handle 11 and the central chamber 23 of the catheter main body 12 and is communicated with the liquid passage of the ablation electrode 17, or may extend to the distal end of the central chamber 23 of the catheter main body 12 through the control handle 11 and is communicated with the first eccentric chamber 35 of the elastic tip tube 31.

The perfusion pipeline 26 may be made of any suitable material, and the distal end of the perfusion pipeline 26 extends into the first eccentric chamber 35 of the elastic tip tube 31 through the central chamber 23 of the catheter main body 12 and is communicated with the through hole 80 in the insert 74. The proximal end of the perfusion pipeline 26 extends into the control handle 11 through the central chamber 23 of the catheter main body 12, and may be fixed in any suitable method known by those skilled in the art. For example, the proximal end of the perfusion pipeline 26 is connected with a section of branch pipe 14, as shown in Fig. 1, wherein the branch pipe 14 extends to the outside of the control handle 11, and the end of the branch pipe 14 is connected and fixed with a Luer taper 15.

The perfusion liquid, which may be any suitable liquid, such as normal saline, enters the perfusion pipeline 26 through the branch pipe 14, enters the cavity 76 of the ablation electrode 17 through the perfusion pipeline 26 and the through hole 80, and flows out of the catheter 10 through the holes in the electrode shell.

The traction wire 21 is preferably made of stainless steel or nickel-titanium alloy. As shown in Fig. 2, Fig. 3 and Fig. 5, the distal end of the traction wire 21 extends into the third eccentric chamber 37 of the elastic tip tube 31 through the central chamber 23, and a spring tube 29 is preferably sleeved outside a section of the traction wire 21 extending in the catheter main body 12 The spring tube 29 is preferably of a tight structure with a tightening force, and a second protective sleeve (not shown in the figure) is sleeved outside the spring tube 29. The second protective sleeve may be made of any suitable material, preferably a polyamide material, and is used for the extension of the spring tube 29 therein. The distal end and proximal end of the second protective sleeve may be fixed on the spring tube 29 by bonding, welding or other suitable manner, such as being bonded on the spring tube 29 with UV cured adhesive. As shown in Fig. 5, a first protective sleeve 32 is preferably sleeved outside a section of traction wire 21 extending in the elastic tip tube 31. The first protective sleeve 32 may be made of any suitable material, preferably a polytetrafluoroethylene material, and is provided in the elastic tip tube 31 and used for the extension of the traction wire 21 therein.

As shown in Fig. 5, the distal end of the traction wire 21 extends into the hole 82 in the insert 74, and the end thereof is fixed by welding, bonding or other suitable manner, preferably being fixed by welding.

The proximal end of the traction wire 21 is fixed on the control handle 11 by adopting any suitable method known by those skilled in the art. For example, a method for fixing a traction wire as disclosed in United States Patent US6120476 may be used. As shown in Fig. 2, Fig. 3 and Fig. 4, the distal end of the conducting wire 25 extends into the second eccentric chamber 36 of the elastic tip tube 31 through the central chamber 23, and is connected with the ablation electrode 17, the ring electrode 16 and the temperature sensor 33 respectively in a manner of welding or other suitable manner, preferably being fixed by welding. Preferably, a conduit 27 is provided outside the conducting wire 25.

The proximal end of the conducting wire 25 is fixed on the control handle 11 by adopting any suitable method known by those skilled in the art, such as being fixed on a corresponding plug by welding.

In a specific implementation of the present invention, during the ablation process of the ablation electrode 17, the nonmetallic heat insulation layer 78 is provided on the through hole 81 of the insert 74, and the temperature sensor 33 may be provided on the inner surface of the electrode shell 71 or in the through hole of the electrode shell 71 or partially provided in the electrode shell, so the temperature detected by the ablation electrode of the present invention is closer to the temperature of the tissue under the same catheter radio-frequency ablation power and perfusion condition commonly used in clinical practice. Wherein, according to a preferred specific implementation of the present invention, the temperature sensor 33 is provided on the inner surface of the electrode shell 71, more preferably, the temperature sensor 33 is partially provided in the electrode shell 71, and even more preferably, the temperature sensor 33 is provided in the through hole of the electrode shell 71.

Fig. 8 is a sectional view of the ablation electrode 17 according to a further implementation of the present invention. As shown in Fig. 8, the ablation electrode 17 includes an electrode shell 71 and a cavity 76 therein, and the insert 74 is provided at the proximal end of the electrode shell 71. The insert 74 may be cylindrical, disc-shaped or in other suitable shape and at least includes a through hole 81, and the distal end of the through hole 81 extends into a through hole 79 of the electrode shell 71. The extending section of the through hole 81 and the insert 74 may be formed integrally or separately. For example, a hollow tube is inserted into the through hole 81, or the distal end of the through hole 81 is connected with a pipeline or other suitable structure. The proximal end of the through hole 81 is opened at the proximal end of the insert 74. The distal end of the through hole 81 turns in the cavity 76, extends into the through hole 79 in the side wall of the electrode shell 71, is opened on the side wall, and is flush with the outer surface of the electrode shell 71, as shown in Fig. 8. A temperature sensor 33 is provided at the distal end of the through hole 81 and is fixed by glue bonding. The insert 74 further includes a through hole 80, and the through hole 80 is a liquid passage for inflow of perfusion liquid. The through hole 80 and the through hole 81 are communicated with the first eccentric chamber 35 and the second eccentric chamber 36 of the elastic tip tube 31 respectively.

A nonmetallic heat insulation layer 78 is provided on the through hole 81. When the extending section of the through hole 81 and the insert 74 are formed integrally and the insert 74 is made of a metallic material, the nonmetallic heat insulation layer 78 is provided on the inner wall and/or outer wall of the through hole 81. When the insert 74 is made of a nonmetallic material, the through hole 81 itself may be used as a nonmetallic heat insulation layer. When the extending section of the through hole 81 and the insert 74 are formed separately and the extending section of the through hole 81 is made of a metallic material, the nonmetallic heat insulation layer 78 is provided on the inner wall and/or outer wall of the through hole 81. When the extending section of the through hole 81 of the insert is made of a nonmetallic material, the through hole 81 itself may be used as a nonmetallic heat insulation layer.

In the embodiment shown in Fig. 8, the rest structures of the catheter 10 are the same as those in the embodiments shown in Fig. 4 and Fig. 5.

Fig. 9 shows a structural diagram of the ablation electrode 17 according to a further implementation of the present invention. The ablation electrode 17 includes a cylindrical side wall surface 77 and a rounded top surface 75, and may be formed separately or integrally. Holes 72 for outflow of the perfusion liquid from the ablation electrode 17 are formed in the rounded top surface 75. Annular grooves 73 are provided in the cylindrical side wall surface 77, and the annular grooves 73 may be provided in a conventional manner for those skilled in the art. For example, the annular grooves 73 may be provided in a manner mentioned in United States Patent US20080294158.

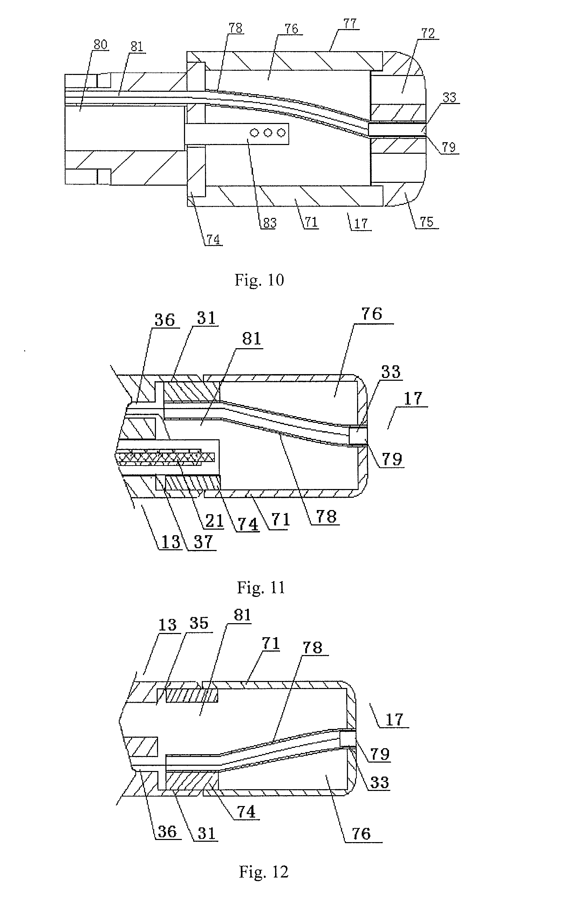

Fig. 10 shows a sectional view of the ablation electrode 17 shown in Fig. 8. The ablation electrode 17 includes a cavity 76, and an insert 74 is provided at the proximal end of the electrode shell 71. The insert 74 is provided at the proximal end of the ablation electrode 17. The insert 74 may be cylindrical, disc-shaped or in other suitable shape and includes a through hole 81, and the distal end of the through hole 81 extends into the through hole 79 of the electrode shell 71. The extending section of the through hole 81 and the insert 74 may be formed integrally or separately. For example, a hollow tube is inserted into the through hole 81, or the distal end of the through hole 81 is connected with a pipeline or other suitable structures. The proximal end of the through hole 81 is opened at the proximal end of the insert 74, and the distal end of the through hole 81 is opened on the outer surface of the electrode shell 71 and is flush with the outer surface of the electrode shell 71. A temperature sensor 33 is provided at the distal end of the through hole 81 and is fixed by glue bonding. The insert 74 further includes a through hole 80, and the through hole 80 is a liquid passage for inflow of perfusion liquid, which may be a central chamber or an eccentric chamber.

The nonmetallic heat insulation layer 78 is provided on the through hole 81. When the extending section of the through hole 81 and the insert 74 are formed integrally and the insert 74 is made of a metallic material, the nonmetallic heat insulation layer 78 is provided on the inner wall and/or outer wall of the through hole 81. When the insert 74 is made of a nonmetallic material, the through hole 81 itself may be used as a nonmetallic heat insulation layer. When the extending section of the through hole 81 and the insert 74 are formed separately and the extending section of the through hole 81 is made of a metallic material, the nonmetallic heat insulation layer 78 is provided on the inner wall and/or outer wall of the through hole 81. When the extending section of the through hole 81 of the insert is made of a nonmetallic material, the through hole 81 itself may be used as a nonmetallic heat insulation layer. The nonmetallic heat insulation layer 78 may completely or partially cover the inner wall and/or outer wall of the through hole 81. Preferably, an extension tube 83 may also be provided at the distal end of the hollow tube in the through hole 80, and may be fixed by glue bonding, welding or other suitable manner. The extension tube 83 and the hollow tube may also be formed integrally. The insert 74 may be completely placed in the cavity of the electrode shell 71, or partially placed in the cavity of the electrode shell 71, as shown in Fig. 10.

In the implementations shown in Fig. 9 and Fig. 10, the rest structures of the catheter 10 may be the same as those in the embodiments shown in Fig. 4 and Fig. 5, or the catheter structures in United States Patent US20080294158.

Fig. 11 shows a sectional view of the ablation electrode 17 according to a further implementation of the present invention; and Fig. 12 shows a sectional view of the ablation electrode 17 in Fig. 11. As shown in Fig. 11 and Fig. 12, the ablation electrode 17 includes an electrode shell 71 and a cavity 76 therein, and the insert 74 is provided at the proximal end of the electrode shell 71. The insert 74 may be circular or in other suitable shape, such as cylindrical, disc-shaped and the like. The insert includes a through hole 81, and the distal end of the through hole 81 extends into the through hole 79 of the electrode shell 71. The extending section of the through hole 81 and the insert 74 may be formed integrally or separately. For example, a hollow tube is inserted into the through hole 81, or the distal end of the through hole 81 is connected with a pipeline or other suitable structures. The proximal end of the through hole 81 is opened at the proximal end of the insert 74. The distal end of the through hole 81 is opened on the outer surface of the electrode shell 71 and is flush with the outer surface of the electrode shell 71. A temperature sensor 33 is provided at the distal end of the through hole 81 and is fixed by glue bonding. Further, a stainless steel tube is welded on the inner wall of the through hole 81, and the distal end of the traction wire 21 extends into the stainless steel tube and is fixed by welding.

As shown in Fig. 11, the perfusion passage is provided with a proximal end and a distal end, wherein the distal end of the perfusion passage extends into the first eccentric chamber 35 of the elastic tip tube 31 through the control handle 11 and the central chamber 23 of the catheter main body 12, and is communicated with the liquid passage of the ablation electrode 17. A perfusion pipeline 26 is provided in the perfusion passage, and the distal end of the perfusion pipeline 26 extends to the distal end of the central chamber 23 of the catheter main body 12 through the control handle 11 and is communicated with the first eccentric chamber 35 of the elastic tip tube 31. There is no perfusion pipeline in the first eccentric chamber 35 of the elastic tip tube 31, and the perfusion liquid directly enters the cavity 76 of the electrode shell 71 from the first eccentric chamber 35 and the through hole 81. The elastic tip tube 31 may also have a structure with four or more chambers, so that the perfusion liquid may flow into the cavity 76 of the electrode shell through at least two eccentric chambers of the elastic tip tube 31.

The distal end of the conducting wire 25 is welded on the stainless steel tube, and a sealant is filled at the distal end of the conducting wire and the proximal end of the through hole 81 to avoid direct contact with the perfusion liquid.

The nonmetallic heat insulation layer 78 is provided on the through hole 81. When the extending section of the through hole 81 and the insert 74 are formed integrally and the insert 74 is made of a metallic material, the nonmetallic heat insulation layer 78 is provided on the inner wall and/or outer wall of the through hole 81. When the insert 74 is made of a nonmetallic material, the through hole 81 itself may be used as a nonmetallic heat insulation layer. When the extending section of the through hole 81 and the insert 74 are formed separately and the extending section of the through hole 81 is made of a metallic material, the nonmetallic heat insulation layer 78 is provided on the inner wall and/or outer wall of the through hole 81. When the extending section of the through hole 81 of the insert is made of a nonmetallic material, the through hole 81 itself may be used as a nonmetallic heat insulation layer.

In the embodiment shown in Fig. 11 and Fig. 12, the rest structure of the catheter 10 is the same as that in the embodiments shown in Fig. 4 and Fig. 5, the embodiment shown in Fig. 8 or the embodiments shown in Fig. 9 and Fig. 10.

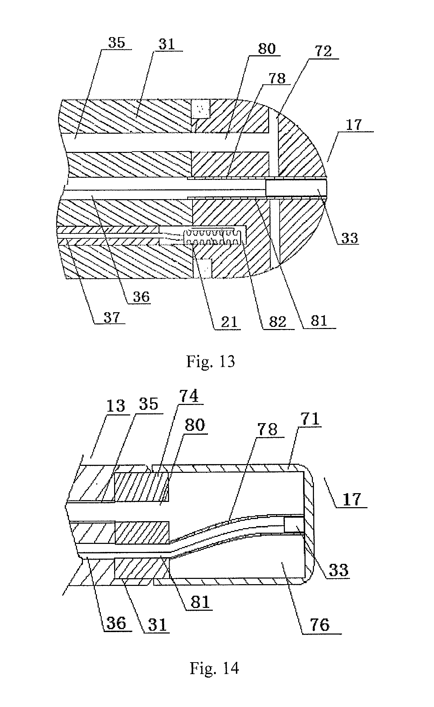

Fig. 13 shows a sectional view of the ablation electrode 17 according to a further implementation of the present invention. As shown in Fig. 13, the ablation electrode 17 includes at least one liquid passage 80, the liquid passage 80 is provided with an opening at the proximal end of the ablation electrode 17, and the perfusion liquid may flow to the outer surface of the ablation electrode 17 through the liquid passage 80. There are multiple ways for the perfusion liquid to flow through the ablation electrode. For example, the liquid passage is provided with a plurality of branches which extend to and are opened on the outer surface of the ablation electrode.

As shown in Fig. 13, the ablation electrode 17 further includes a through hole 81, and the distal end of the through hole 81 is opened on the outer surface of the electrode shell 71 and is flush with the outer surface of the electrode shell 71. The temperature sensor 33 is provided at the distal end of the through hole 81 and is fixed by glue bonding. The ablation electrode 17 further includes a blind hole 82 in which the traction wire 21 is fixed. The through hole 80, the through hole 81 and the blind hole 82 are communicated with the first eccentric chamber 35, the second eccentric chamber 36 and the third eccentric chamber 37 of the elastic tip tube 31 respectively. The nonmetallic heat insulation layer 78 is completely or partially provided on the inner wall of the through hole 81. The nonmetallic heat insulation layer 78 may be a tubular heat insulation layer inserted into the through hole 81, or may be a nonmetallic heat insulation layer coated on the inner wall of the through hole 81. The nonmetallic heat insulation layer 78 is made of a material with a heat insulation function, and may be made of a nonmetallic material such as a high polymer material, ceramic or other suitable nonmetallic material. The nonmetallic heat insulation layer 78 may be made of a nonmetallic material with high temperature resistance. The liquid passage 80 may also be completely or partially provided with the nonmetallic heat insulation layer 78 therein.

In the implementation shown in Fig. 13, the rest structures of the catheter 10 are the same as those in the implementations shown in Fig. 4 and Fig. 5 or the catheter structures in Chinese Patent CN201020215408.8.

Fig. 14 is a sectional view of the ablation electrode 17 according to a further implementation of the present invention.

As shown in Fig. 14, the ablation electrode 17 includes an electrode shell 71 and a cavity 76 therein, and an insert 74 is provided at the proximal end of the electrode shell 71. The insert 74 may be cylindrical, disc-shaped or in other suitable shape and at least includes a through hole 81, and the distal end of the through hole 81 extends into a through hole 79 of the electrode shell 71. The extending section of the through hole 81 and the insert 74 may be formed integrally or separately. For example, a hollow tube is inserted into the through hole 81, or the distal end of the through hole 81 is connected with a pipeline or other suitable structure. The proximal end of the through hole 81 is opened at the proximal end of the insert 74. The distal end of the through hole 81 extends to the inner surface of the electrode shell 71 and is flush with the inner surface of the electrode shell 71, or may partially extend into the electrode shell 71. A temperature sensor 33 is provided at the distal end of the through hole 81 and is fixed by glue bonding. In this implementation, when the insert 74 and the extending section of the through hole 81 are separated structures, a hollow tube may be provided in the through hole 81. The distal end of the hollow tube extends to the inner surface of the electrode shell 71 and is flush with the inner surface of the electrode shell 71, or may partially extend into the electrode shell 71. The distal end of the hollow tube has a closed construction. The temperature sensor 33 is glue bonded and fixed at the distal end of the hollow tube. The thickness of the electrode shell where the temperature sensor is located is less than 0.2mm. Preferably, the thickness of the electrode shell where the temperature sensor is located is less than 0.1mm.

The insert 74 further includes a through hole 80, and the through hole 80 is a liquid passage for inflow of perfusion liquid. The through hole 80 and the through hole 81 are communicated with the first eccentric chamber 35 and the second eccentric chamber 36 of the elastic tip tube 31 respectively.

A nonmetallic heat insulation layer 78 is provided on the through hole 81. When the extending section of the through hole 81 and the insert 74 are formed integrally and the insert 74 is made of a metallic material, the nonmetallic heat insulation layer 78 is provided on the inner wall and/or outer wall of the through hole 81. When the insert 74 is made of a nonmetallic material, the through hole 81 itself may be used as a nonmetallic heat insulation layer. When the extending section of the through hole 81 and the insert 74 are formed separately and the extending section of the through hole 81 is made of a metallic material, the nonmetallic heat insulation layer 78 is provided on the inner wall and/or outer wall of the through hole 81. When the extending section of the through hole 81 of the insert is made of a nonmetallic material, the through hole 81 itself may be used as a nonmetallic heat insulation layer.

The temperature sensor 33 and a part of the electrode shell which is not directly cooled by the perfusion liquid may be in direct contact, or may be connected through a metallic material, such as welding, or connected through a nonmetallic heat-conducting material, such as bonding through a heat-conducting glue, or connected through both a metallic material and a nonmetallic heat-conducting material, or connected through other suitable connecting methods. Therefore, the heat conductivity between the temperature sensor 33 and the outer wall of the electrode shell 71 is better than that between the temperature sensor 33 and the liquid passage. The part of the electrode shell which is not directly cooled by the perfusion liquid in the present invention refers to such a part on the electrode shell that is not in direct contact with the perfusion liquid. The liquid passage in the present invention includes a part through which the perfusion liquid flows in the ablation electrode 17, including the hole 80, the cavity 76 of the electrode shell 17 and the part on the electrode shell for outflow of the perfusion liquid.

In the embodiment shown in Fig. 14, the rest structures of the catheter 10 are the same as those in the embodiment shown in Fig. 4 and Fig. 5, the embodiment shown in Fig. 9 and Fig. 10, the embodiment shown in Fig. 11 and Fig. 12 or other embodiments. The implementations of the present invention are not limited to the above-mentioned embodiments, and in order to meet other requirements of the liquid passages or requirements of mechanical structures, the form of the electrode body and/or the insert may be changed (e.g. the diameter is changed, the number of passages is adjusted, and the length is changed), so as to meet the requirements upon cooling and temperature measuring during ablation and the requirements upon the connection between the ablation electrode and the catheter main body. Various variations and improvements of the present invention in forms and details may be made by those skilled in the art without departing from the spirit and scope of the present invention, all of which are considered to fall into the protection scope of the present invention.

## Claims

1. An ablation electrode for a catheter, **characterized in** comprising an electrode shell, and an optional cavity and a temperature sensor in the electrode shell, wherein a liquid passage for outflow of perfusion liquid is provided in the electrode shell, and a liquid passage for inflow of the perfusion liquid is provided in the proximal end of the electrode shell; and a heat-conducting insulation structure is provided between the temperature sensor and the liquid passages, and the thickness of the electrode shell where the temperature sensor is located is less than 0.2mm.

2. The ablation electrode of claim 1, **characterized in that** the electrode shell is provided with a through hole, and the temperature sensor is provided in the through hole.

3. The ablation electrode of claim 1 or 2, **characterized in that** an insert is provided at the proximal end of the electrode shell, the distal end of the insert extends into the cavity, and the insert comprises at least one through hole; the distal end of the through hole in the insert extends into the through hole of the electrode shell, the proximal end of the through hole in the insert is opened at the proximal end of the insert, the distal end of the through hole in the insert is opened on the outer surface of the electrode shell and is flush with the outer surface of the electrode shell, and the temperature sensor is provided at the distal end of the through hole in the insert.

4. The ablation electrode of claim 2 or 3, **characterized in that** the diameter of the through hole in the electrode shell is less than 1mm; and preferably, the diameter of the through hole in the electrode shell is less than 0.5mm.

5. The ablation electrode of claim 1, **characterized in that** an insert is provided at the proximal end of the electrode shell, the distal end of the insert extends into the cavity, and the insert comprises at least one through hole; the distal end of the through hole in the insert extends to the inner surface of the electrode shell and is flush with the inner surface of the electrode shell, or the distal end of the through hole in the insert partially extends into the electrode shell; the proximal end of the through hole in the insert is opened at the proximal end of the insert, the distal end of the through hole in the insert is opened or has a closed construction, and the temperature sensor is provided at the distal end of the through hole in the insert; and preferably, the thickness of the electrode shell where the temperature sensor is located is less than 0.1 mm.

6. The ablation electrode of any of the claims above, **characterized in that** the heat-conducting insulation structure is a nonmetallic heat insulation layer.

7. The ablation electrode of claim 6, **characterized in that** the nonmetallic heat insulation layer is provided on the inner wall and/or outer wall of the through hole in the insert; and preferably, the inner wall and/or outer wall of the through hole in the insert is partially or completely provided with the nonmetallic heat insulation layer.

8. The ablation electrode of any of the claims above, **characterized in that** the distance between a temperature sensing portion of the temperature sensor and the top end of the temperature sensor is less than 0.5mm; preferably, the distance between the temperature sensing portion of the temperature sensor and the top end of the temperature sensor is less than 0.2mm; and more preferably, the distance between the temperature sensing portion of the temperature sensor and the top end of the temperature sensor is less than 0.1mm.

9. The ablation electrode of any of the claims above, **characterized in that** the nonmetallic heat insulation layer is made of a high polymer material or ceramic.

10. A perfused electrode catheter, **characterized in** comprising:
a catheter main body having a proximal end, a distal end and a central chamber penetrating through the catheter main body;
an ablation portion, comprising a section of elastic tip tube, and having a proximal end, a distal end and at least one chamber penetrating through ablation portion, the proximal end of the ablation portion is fixedly connected with the distal end of the catheter main body;
an ablation electrode, fixedly connected to the distal end of the ablation portion, and comprising an electrode shell, and an optional cavity and a temperature sensor in the electrode shell, wherein a liquid passage for outflow of perfusion liquid is provided in the electrode shell, and a liquid passage for inflow of the perfusion liquid is provided in the proximal end of the electrode shell; and a heat-conducting insulation structure is provided between the temperature sensor and the liquid passages, and the thickness of the electrode shell where the temperature sensor is located is less than 0.2mm;
a perfusion passage, having a proximal end and a distal end, the distal end of the perfusion passage extends into the chamber of the ablation portion through the central chamber of the catheter main body and is communicated with the liquid passage of the ablation electrode.

11. The perfused electrode catheter of claim 10, **characterized in that** the electrode shell is provided with a through hole, and the temperature sensor is provided in the through hole.

12. The perfused electrode catheter of claim 10 or 11, **characterized in that** an insert is provided at the proximal end of the electrode shell, the distal end of the insert extends into the cavity, and the insert comprises at least one through hole; the distal end of the through hole in the insert extends into the through hole of the electrode shell, the proximal end of the through hole in the insert is opened at the proximal end of the insert, the distal end of the through hole in the insert is opened on the outer surface of the electrode shell and is flush with the outer surface of the electrode shell, and the temperature sensor is provided at the distal end of the through hole in the insert.

13. The perfused electrode catheter of claim 11 or 12, **characterized in that** the diameter of the through hole in the electrode shell is less than 1mm; and preferably, the diameter of the through hole in the electrode shell is less than 0.5mm.

14. The perfused electrode catheter of claim 10, **characterized in that** an insert is provided at the proximal end of the electrode shell, the distal end of the insert extends into the cavity, and the insert comprises at least one through hole; the distal end of the through hole in the insert extends to the inner surface of the electrode shell and is flush with the inner surface of the electrode shell, or he distal end of the through hole in the insert partially extends into the electrode shell; the proximal end of the through hole in the insert is opened at the proximal end of the insert, the distal end of the through hole in the insert is opened or has a closed construction, and the temperature sensor is provided at the distal end of the through hole in the insert; and preferably, the thickness of the electrode shell where the temperature sensor is located is less than 0.1 mm.

15. The perfused electrode catheter of any of the claims above, **characterized in that** a perfusion pipeline is provided in the perfusion passage, and the distal end of the perfusion pipeline may extend to the distal end of the chamber of the ablation portion through a control handle and the central chamber of the catheter main body and is communicated with the liquid passage of the ablation electrode, or extend to the distal end of the central chamber of the catheter main body through the control handle and is communicated with at least one chamber penetrating through the ablation portion.

16. The perfused electrode catheter of any of the claims above, **characterized in that** the heat-conducting insulation structure is a nonmetallic heat insulation layer.

17. The perfused electrode catheter of claim 16, **characterized in that** the nonmetallic heat insulation layer is provided on the inner wall and/or outer wall of the through hole in the insert; and preferably, the inner wall and/or outer wall of the through hole in the insert is partially or completely provided with the nonmetallic heat insulation layer.

18. The perfused electrode catheter of any of the claims above, **characterized in that** the distance between a temperature sensing portion of the temperature sensor and the top end of the temperature sensor is less than 0.5mm; preferably, the distance between the temperature sensing portion of the temperature sensor and the top end of the temperature sensor is less than 0.2mm; and more preferably, the distance between the temperature sensing portion of the temperature sensor and the top end of the temperature sensor is less than 0.1mm.

19. The perfused electrode catheter of any of the claims above, **characterized in that** the nonmetallic heat insulation layer is made of a high polymer material or ceramic.
